# FASCICULE DE BREVET EUROPEEN

(11) **EP 1 306 078 B1**
(45) Date de publication et mention de la délivrance du brevet: **06.09.2006**
(21) Numéro de dépôt: 02292627.3
(22) Date de dépôt: 23.10.2002
(51) Int. Cl.: A61K 8/87, A61Q 5/06

(54) **Composition de traitement cosmétique de cheveux comprenant un polyuréthane fixant non associatif et un polyuréthane associatif anionique ou non ionique, et procédé de traitement cosmétique**
Kosmetische Zusammensetzung zur Haarbehandlung, die ein haarfixierendes nichtassoziatives Polyurethan und ein anionisches oder nichtionisches assoziatives Polyurethan enthält und kosmetisches Behandlungsverfahren
Cosmetic hair treatment composition comprising a nonassociative hair-fixing polyurethane and an anionic or nonionic associative polyurethane, and cosmetic treatment process

(30) Priorité: 26.10.2001 FR 0113904
(43) Date de publication de la demande: 02.05.2003
(60) Demande divisionnaire: 05026020.7
(73) Titulaire: L'ORÉAL, 75008 Paris (FR)
(72) Inventeur: Pataut, Francoise, 75017 Paris (FR); Gringore, Charles, 75017 Paris (FR)
(74) Mandataire: Dossmann, Gérard

(56) Documents cités:
- EP-A- 1 062 935
- EP-A- 1 132 076
- WO-A-01/54660
- FR-A- 2 774 899
- US-A- 6 080 392

## Description

La présente invention est relative à une composition de traitement cosmétique des cheveux contenant en association, au moins un polyuréthane fixant non associatif et au moins un polyuréthane associatif anionique ou non ionique, et conditionnée dans un dispositif aérosol, ainsi qu'à un procédé de traitement cosmétique mettant en oeuvre cette composition.

Les produits coiffants, tels que des laques, des mousses et des gels, sont bien connus dans la technique et sont habituellement utilisés pour structurer la coiffure et lui apporter une tenue durable.

Les produits coiffants contiennent généralement des polymères fixants anioniques ou non ioniques dans un milieu cosmétiquement acceptable.

Le document US-A-6 080 392 décrit une composition cosmétique en aérosol, comprenant au moins un polymère polyuréthane associatif et un polymère fixant anionique.

Cependant, certains polymères entraînent un durcissement de la chevelure. Les cheveux sont alors souvent collés entre eux et la coiffure est figée.

La demanderesse a trouvé de manière surprenante qu'en associant un polyuréthane fixant non associatif et un polyuréthane associatif anionique ou non ionique en milieu aérosol, on obtenait une mise en forme de la coiffure par un simple travail aux doigts et que cette association permettait de surmonter les inconvénients tels que décrits ci-dessus, rencontrés avec les produits habituels de fixation.

Cette composition présente en outre l'avantage de bien s'étaler sur les cheveux et l'application peut se faire aussi bien sur cheveux secs que sur cheveux humides.

Dans le cas d'une application sur cheveux humides, un séchage à l'air libre ou au sèche-cheveux peut être réalisé. Le résultat est une coiffure souple et naturelle.

L'invention a donc pour objet une composition de traitement cosmétique des cheveux telle que décrite ci-dessous.

Un autre objet de la présente invention est un procédé de traitement cosmétique des cheveux mettant en oeuvre la composition selon l'invention.

L'invention a encore pour objet l'utilisation de la composition selon l'invention comme produit de coiffage.

D'autres objets, caractéristiques, aspects et avantages de l'invention apparaîtront encore plus clairement à la lecture de la description et des divers exemples qui suivent.

La présente invention concerne une composition de traitement cosmétique des cheveux comprenant, dans un milieu cosmétiquement acceptable, au moins un polyuréthane fixant non associatif et au moins un polyuréthane associatif anionique ou non ionique, conditionnée dans un dispositif aérosol en présence d'un agent propulseur.

Par milieu cosmétiquement acceptable, on entend un milieu compatible avec les cheveux, mais aussi d'odeur, d'aspect et de toucher agréables.

Par polyuréthane fixant non associatif, on entend au sens de la présente invention, des polycondensats comprenant au moins une séquence polyuréthanne susceptible d'apporter du maintien à la coiffure et qui ne comprennent pas dans leur structure de chaîne grasse terminale ou pendante comportant plus de 10 atomes de carbone. Ils sont décrits en particulier dans les brevets EP 0 751 162, EP 0 637 600, FR 2 743 297 et EP 0 648 485 dont la Demanderesse est titulaire, ainsi que les brevets EP 0 656 021 ou WO 94/03510 de la Société BASF et EP 0 619 111 de la Société National Starch.

Les polyuréthanes fixants non associatifs utilisés conformément à l'invention peuvent être solubles dans le milieu cosmétiquement acceptable, notamment après neutralisation par une base organique ou minérale, ou encore former une dispersion dans ce milieu. La dispersion peut comprendre alors au moins 0,05 % de tensioactif permettant la mise en dispersion et le maintien en dispersion du polyuréthane fixant non associatif.

Selon l'invention, on peut utiliser tout type de tensioactif dans ladite dispersion, mais de préférence un tensioactif non ionique. La taille moyenne des particules du polyuréthane fixant non associatif dans la dispersion est de préférence comprise entre 0,1 et 1 micron.

A titre d'exemple, le polyuréthane fixant non associatif peut être formé par un arrangement de blocs, cet arrangement étant obtenu notamment à partir de :
(1) au moins un composé qui contient deux ou plus de deux atomes d'hydrogène actifs par molécule ;
(2) au moins un diol ou un mélange de diols contenant des fonctions acides ou leurs sels ; et
(3) au moins un di- ou polyisocyanate.

Avantageusement, les composés (1) sont choisis dans le groupe comprenant les diols, les diamines, les polyesterols, les polyétherols ou leurs mélanges.

Les composés (1) qui sont préférés sont les polyéthylèneglycols et les polypropylèneglycols linéaires, en particulier ceux qui sont obtenus par réaction de l'oxyde d'éthylène ou de propylène avec l'eau ou du diéthylène ou du dipropylèneglycol en présence d'hydroxyde de sodium en tant que catalyseur. Ces polyalkylèneglycols ont généralement une masse moléculaire comprise entre environ 600 et 20 000.

D'autres composés organiques préférés sont ceux qui ont des groupes mercapto, amino, carboxyle ou hydroxyle. Parmi ceux-ci, on cite plus particulièrement les composés polyhydroxylés tels que les polyéther-diols, les polyester-diols, les polyacétal-diols, les polyamide-diols, les polyester-polyamide-diols, les poly(alkylène éther)-diols, les polythioéther-diols et les polycarbonate-diols.

Les polyéther-diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats d'oxyde d'alkylène. Des polyéthers appropriés sont par exemple préparés par condensation d'oxydes d'alkylène et d'alcools polyhydriques, tels que l'éthylèneglycol, le 1,2-propylèneglycol et le 1,4-butanediol.

Les polyester-diols, polyester-amides, polyamide-diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylèneglycol, le 1,2-propylèneglycol, le 1,4-butanediol, le néopentylglycol et l'hexanediol. On peut aussi utiliser des aminoalcools, par exemple l'éthanolamine. Des diamines appropriées pour préparer les amide-polyesters sont l'éthylène-diamine et l'hexaméthylène-diamine.

Des polyacétals appropriés peuvent être préparés, par exemple, à partir de 1,4-butanediol ou d'hexanediol et de formaldéhyde. Des polythioéthers appropriés peuvent être préparés par exemple par réaction de condensation entre des thioglycols seuls ou en combinaison avec d'autres glycols tels que l'éthylèneglycol, le 1,2-propylèneglycol ou avec d'autres composés polyhydroxylés. Les composés polyhydroxylés contenant déjà des groupements uréthannes, des polyols naturels, qui peuvent être davantage modifiés, par exemple, l'huile de castor et les carbohydrates peuvent également être utilisés.

Plus préférentiellement, le composé du groupe (1) est un polyestérol, notamment un polyester-diol formé par la réaction d'au moins un (di)-polyol (1ₐ) et d'au moins un acide (1_{b}). Le (di)- polyol (1ₐ) est en particulier choisi dans le groupe comprenant le néopentylglycol, le 1,4-butanediol, l'hexanediol, l'éthylèneglycol, le diéthylèneglycol, le propylèneglycol, le butylèneglycol, le néopentylglycol et (di)-polyéthylèneglycol. L'acide (1_{b}) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol-propanoïque (DMPA) ou un acide 2,2-hydroxyméthyl-carboxylique. En général, le composé (2) est utile en tant que bloc de couplage. En tant que composés (2), on préfère ceux comprenant au moins un poly (acide-(alpha-hydroxycarboxyliquediol)).

Les composés (2) particulièrement préférés conformément à l'invention sont ceux choisis dans le groupe comprenant l'acide 2,2-di-(hydroxyméthyl)acétique, l'acide 2,2-dihydroxyméthylpropionique, l'acide 2,2-dihydroxyméthylbutyrique, l'acide 2,2-dihydroxyméthylpentanoïque.

Le di- ou polyisocyanate (3) peut être choisi en particulier dans le groupe comprenant l'hexaméthylène-diisocyanate, l'isophorone-diisocyanate (IDPI), le toluylène-diisocyanate, le diphénylméthane-4,4'-diisocyanate (DPMD) et le dicyclohexylméthane-4,4'-diisocyanate (DCMD), le méthylène-di-p-phényl-diisocyanate, le méthylène-bis(4-cyclohexylisocyanate), les toluène-diisocyanates, le 1,5-naphtalène-diisocyanate, le 4,4'-diphénylméthane-diisocyanate, le 2,2'-diméthyl-4,4'-diphénylméthane-diisocyanate, le 1,3-phénylène-diisocyanate, le 1,4-phénylène diisocyanate, des mélanges de 2,4- et de 2,6-toluène-diisocyanates, le 2,2'-dichloro-4,4'-diisocyanato-diphénylméthane, le 2,4-dibromo-1,5-diisocyanato naphtalène, le butane-1,4-diisocyanate, l'hexane-1,6-diisocyanate, le cyclohexane-1,4-diisocyanate.

Le polyuréthane fixant non associatif peut être formé à l'aide d'un composé supplémentaire (4) servant en général à allonger sa chaîne. Ces composés (4) peuvent être choisis dans le groupe comprenant notamment les glycols saturés ou insaturés tels que l'éthylèneglycol, le diéthylèneglycol, le néopentylglycol, le triéthylène glycol ; les aminoalcools tels que l'éthanolamine, la propanolamine, la butanolamine ; les amines primaires hétérocycliques, aromatiques, cycloaliphatiques, et aliphatiques ; les diamines ; les acides carboxyliques tels que les acides carboxyliques aliphatiques, aromatiques et hétérocycliques comme les acides oxalique, succinique, glutarique, adipique, sébacique et téréphtalique ; les acides aminocarboxyliques. Les composés (4) préférés sont les diols aliphatiques.

Les polyuréthanes fixants non associatifs utilisés selon l'invention peuvent également être formés à partir de composés supplémentaires (5) ayant un squelette siliconé tels que les polysiloxanes, les polyalkylsiloxanes ou les polyarylsiloxanes, notamment les polyéthylsiloxanes, les polyméthylsiloxanes et les polyphénylsiloxanes, comportant éventuellement des chaînes hydrocarbonées greffées sur les atomes de silicium.

Les polyuréthanes fixants non associatifs utilisés avantageusement comprennent un motif répétitif de base répondant à la formule générale (I) :

- O - B - O - CO - NH - R - NH - CO - (I)

dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes alkylènes de type aromatique, aliphatique en C₁ à C₂₀, cycloaliphatique en C₁ à C₂₀, ces groupes étant substitués ou non.

Le groupe R est avantageusement choisi parmi les groupes répondant aux formules suivantes :

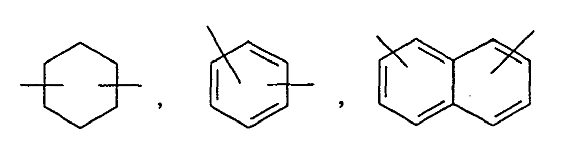

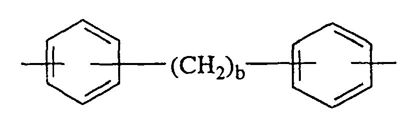

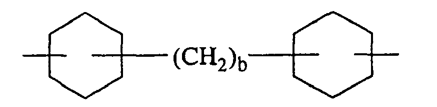

―(CH₂)_{c}―

dans lesquelles b est un nombre entier compris entre 0 et 3, et c un nombre entier compris entre 1 et 20, de préférence compris entre 2 et 12.

En particulier, le groupe R est choisi parmi les groupes hexaméthylène, 4,4'-biphénylèneméthane, 2,4- et/ou 2,6-tolylène, 1,5-naphtylène, p-phénylène, méthylène- 4,4-bis-cyclohexyle et le groupe divalent dérivé de l'isophorone.

Le polyuréthane fixant non associatif utilisé dans la présente invention peut avantageusement comprendre en outre au moins une séquence polysiloxane dont le motif répétitif de base répond par exemple à la formule générale (II) :

- O - P - O - CO - NH - R - NH - CO - (II)

dans laquelle :
- P est un segment polysiloxanique, et
- R est un groupe divalent choisi parmi les groupes alkylènes de type aromatique, aliphatique en C₁ à C₂₀, cycloaliphatique en C₁ à C₂₀, ces groupes étant substitués ou non.

Avantageusement, le segment polysiloxanique P répond à la formule générale (III) ci-après : 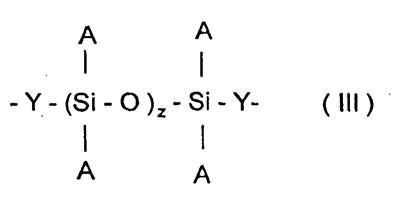 dans laquelle:
- les groupes A, qui peuvent être identiques ou différents, sont choisis parmi d'une part les groupes hydrocarbonés monovalents en C₁ à C₂₀ exempts ou substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne du segment polysiloxane soit comprise entre 300 et 10 000.

En général, le groupe divalent Y est choisi parmi les groupes alkylènes de formule -(CH₂)ₐ-, dans laquelle a représente un nombre entier pouvant être compris entre 1 et 10.

Les groupes A peuvent être choisis parmi les groupes alkyles, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle ; les groupes arylalkyle, notamment benzyle et phényléthyle, ainsi que les groupes tolyle et xylyle.

A titre d'exemples de polyuréthane fixant non associatif, on peut notamment citer le copolymère acide diméthylolpropionique/isophorone-diisocyanate/néopentylglycol/polyesterdiols (connu aussi sous le nom de polyuréthane-1, appellation INCI) vendu sous la marque Luviset® PUR par la société BASF, et le copolymère acide diméthylolpropionique/isophorone-diisocyanate/néopentylglycol/polyesterdiols/diamine siliconée (connu aussi sous le nom de polyuréthane-6, appellation INCI) vendu sous la marque Luviset® Si PUR A par la société BASF.

Les polyuréthanes fixants non associatifs sont notamment utilisés en une quantité de 0,1 à 20 % en poids, de préférence de 0,5 à 10 % en poids, et mieux encore de 1 à 5 % en poids par rapport au poids total de la composition de traitement des cheveux.

Par polyuréthane associatif, on entend un polyuréthane possédant au moins une chaîne grasse terminale ou pendante comportant au moins 10 atomes de carbone. Ce type de polymère est susceptible d'interagir avec lui-même ou avec des composés particuliers tels que des tensioactifs pour conduire à un épaississement du milieu.

A titre d'exemple de polyuréthane associatif anionique, on peut notamment citer un terpolymère acrylique soluble ou gonflable dans les alcalis. Il est caractérisé par le fait qu'il comprend :
a) environ 20 à 70% en poids, de préférence 25 à 55% en poids, d'un acide carboxylique à insaturation α, β-monoéthylénique ;
b) environ 20 à 80% en poids, de préférence 30 à 65% en poids, d'un monomère à insaturation monoéthylénique non tensioactif différent de a) et
c) environ 0,5 à 60% en poids, de préférence 10 à 50% en poids, d'un monomère uréthane non-ionique qui est le produit de réaction d'un tensioactif non-ionique monohydrique avec un monoisocyanate à insaturation monoéthylénique.

L'acide carboxylique à insaturation α, β-monoéthylénique a) peut être choisi parmi de nombreux acides et en particulier l'acide acrylique, l'acide méthacrylique, l'acide itaconique et l'acide maléique. L'acide méthacrylique est préféré. Une large proportion d'acide est essentielle pour donner une structure polymère qui se solubilise et donne un épaississant par réaction avec un composé alcalin comme l'hydroxyde de sodium, les alcanolamines, l'aminométhylpropanol ou l'aminométhylpropanediol.

Le terpolymère doit aussi contenir une proportion importante indiquée ci-dessus d'un monomère b) à insaturation monoéthylénique qui n'a pas de propriété tensioactive. Les monomères préférés sont ceux qui donnent des polymères insolubles dans l'eau lorsqu'ils sont homopolymérisés et sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont les (méth)acrylates de méthyle et d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Cependant, des monomères qui contiennent des groupes réactifs sous l'action de la chaleur peuvent être utilisés dans certaines situations, comme l'acrylate d'hydroxyéthyle.

Les tensioactifs non-ioniques monohydriques utilisés pour obtenir le monomère uréthane non-ionique c) sont bien connus et sont généralement des composés hydrophobes alcoxylés contenant un oxyde d'alkylène formant la partie hydrophile de la molécule. Les composés hydrophobes sont généralement constitués par un alcool aliphatique ou un alkylphénol dans lesquels une chaîne carbonée contenant au moins six atomes de carbone constitue la partie hydrophobe du tensio-actif.

Les tensioactifs non-ioniques monohydriques préférés ont pour formule : 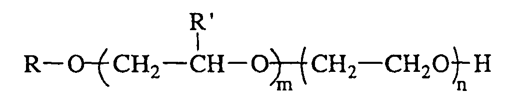 dans laquelle R est un groupe alkyle en C₆-C₃₀ ou aralkyle en C₈-C₃₀, R' est un groupe alkyle en C₁-C₄, n est un nombre moyen allant d'environ 5 à 150 et m est un nombre moyen allant d'environ 0 à 50, à la condition que n soit au moins aussi grand que m et que n + m = 5-150.

A titre de groupes alkyle en C₆-C₃₀ préférés, on peut citer les radicaux dodécyle et alkyle en C₁₈-C₂₆. A titre de groupes aralkyle, on peut citer plus particulièrement les groupes alkyl (C₈-C₁₃) phényle. Le groupe R' préféré est le groupe méthyle.

Le monoisocyanate à insaturation monoéthylénique utilisé pour former le monomère uréthane non-ionique c) peut être choisi parmi des composés très variés. On peut utiliser un composé contenant toute insaturation copolymérisable telle qu'une insaturation acrylique ou méthacrylique. On peut aussi utiliser une insaturation allylique conférée par l'alcool allylique. Les monoisocyanates monoéthyléniques préférés sont l'α,α-diméthyl-m-isopropényl-benzylisocyanate et le méthylstyrène-isopropylisocyanate.

Le terpolymère acrylique défini ci-dessus est obtenu par copolymérisation en émulsion aqueuse des composants a), b) et c) qui est tout à fait usuelle et décrite dans la demande de brevet EP-A-0 173 109.

Comme exemples de polyuréthane associatif anionique pouvant être utilisés selon la présente invention, on peut notamment citer les copolymères d'acide méthacrylique ou acrylique comprenant au moins un motif de (méth)acrylate d'alkyle en C₁₋₃₀ et un motif uréthane substitué par une chaîne grasse. On peut en particulier citer le copopolymère acide méthacrylique/méthacrylate de méthyle/ méthylstyrène-isopropylisocyanate/alcool béhénylique polyéthoxylé (comportant 40 motifs éthoxy) vendu sous la marque Viscophobe® DB 1000 vendu par la société Union Carbide.

Les polyuréthanes associatifs non ioniques utilisés dans la présente invention sont notamment des polyuréthanes-polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers-polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers-polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers-polyuréthanes non-ioniques à chaîne grasse peuvent être des copolymères triblocs dont la séquence hydrophile est une chaîne polyoxyéthoxylée comportant de 50 à 1000 groupements éthoxylés. Les polyéthers-polyuréthanes non-ioniques comportent une liaison uréthanne entre les séquences hydrophiles, d'où l'origine du nom.

Par extension figurent aussi parmi les polyéthers-polyuréthanes non-ioniques à chaîne grasse, ceux dont les séquences hydrophiles sont liées aux séquences lipophiles par d'autres liaisons chimiques.

A titre d'exemples de polyéthers-polyuréthanes non-ioniques à chaîne grasse utilisables dans l'invention, on peut mentionner aussi le Rhéolate 205 à fonction urée vendu par la société RHEOX ou encore les Rhéolates 208, 204 ou 212, ainsi que l'Acrysol® RM 184.

On peut également citer le produit ELFACOS T210 à chaîne alkyle en C₁₂₋₁₄ et le produit ELFACOS® T212 à chaîne alkyle en C₁₈ de chez AKZO.

Le produit DW 1206B de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers-polyuréthanes utilisables selon l'invention sont en particulier ceux décrits dans l'article de G. Fonnum, J. Bakke et Fk. Hansen - Colloid Polym. Sci 271, 380.389 (1993).

Comme exemples préférés de polyuréthane associatif non ionique, on peut citer les polyéthers-polyuréthanes susceptibles d'être obtenus par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther-polyuréthanes sont vendus notamment par la société ROHM & HAAS sous les appellations Aculyn® 46 et Aculyn® 44. L'ACULYN® 46 est un polycondensat de polyéthylèneglycol à 150 ou 180 moles d'oxyde d'éthylène, d'alcool stéarylique et de méthylène-bis(4-cyclohexylisocyanate) (SMDI), à 15% en poids dans une matrice de maltodextrine (4%) et d'eau (81%); l'ACULYN® 44 est un polycondensat de polyéthylèneglycol à 150 ou 180 moles d'oxyde d'éthylène, d'alcool décylique et de méthylène-bis(4-cyclohexylisocyanate) (SMDI), à 35% en poids dans un mélange de propylèneglycol (39%) et d'eau (26%).

Les polyuréthanes associatifs anioniques ou non ioniques sont notamment utilisés en une quantité de 0,1 à 10 % en poids, de préférence de 0,2 à 8 % en poids, et mieux encore de 0,5 à 5 % en poids par rapport au poids total de la composition de traitement des cheveux.

On peut utiliser dans la présente invention tous les agents propulseurs bien connus dans la technique tels que les gaz hydrocarbonés, comme les alcanes en C₃₋₅, par exemple, le propane, le n-butane, l'isobutane ; les gaz fluorés comme par exemple, le chlorodifluorométhane, le dichlorodifluorométhane, le 1,1-difluoroéthane, le chlorodifluoroéthane, le dichlorotétrafluoroéthane ; l'azote, l'air et le dioxyde de carbone ; le diméthyléther ; et leurs mélanges.

De préférence, on utilise le diméthyléther, les gaz hydrocarbonés, ou leurs mélanges tels que, par exemple, les mélanges de diméthyléther et d'alcanes en C₃₋₅.

Les agents propulseurs sont notamment utilisés en une quantité de 2 à 90 % en poids, de préférence de 5 à 80 % en poids par rapport au poids total de la composition de traitement des cheveux.

Le milieu cosmétiquement acceptable peut être constitué uniquement par de l'eau ou par un mélange d'eau et d'un solvant cosmétiquement acceptable tel qu'un alcool inférieur en C₁-C₄, tel que l'éthanol, l'isopropanol, le tertio-butanol, le n-butanol ; les alkylènepolyols comme le propylèneglycol ; les éthers de polyols ; et leurs mélanges.

La composition selon l'invention peut comprendre en outre des additifs classiques bien connus dans la technique, tels que d'autres polymères fixants différents de ceux décrits ci-dessus, les polymères cationiques, amphotères ou zwitteroniques, d'autres polymères anioniques ou non-ioniques différents de ceux décrits ci-dessus, les agents épaississants, les nacrants, les opacifiants, les filtres UV, les sucres, les parfums, les huiles minérales, végétales et/ou synthétiques, les esters d'acides gras, les colorants, les silicones volatiles ou non, organomodifiées ou non, cycliques ou acycliques, ramifiées ou non, les particules minérales ou organiques, naturelles ou synthétiques, les conservateurs et les agents de stabilisation du pH.

L'homme du métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de traitement des cheveux conformes à l'invention peuvent se présenter sous forme d'une mousse, d'un gel, d'un spray ou d'une laque et être utilisées en application rincée ou non. Elles sont conditionnées dans un dispositif aérosol usuel en cosmétique.

Les compositions conformes à l'invention peuvent être utilisées en tant que produits de fixation et/ou de maintien de la coiffure, compositions de soin de cheveux, shampoings, compositions de conditionnement des cheveux, telles que des compositions destinées à apporter de la douceur aux cheveux, ou encore des compositions de maquillage des cheveux.

La présente invention concerne également un procédé de traitement cosmétique des cheveux qui consiste à appliquer une quantité efficace d'une composition telle que décrite ci-dessus, sur les cheveux, à rincer ou non après un éventuel temps de pose.

Selon un mode de réalisation préféré de l'invention, la composition peut être utilisée comme produit de coiffage non rincé.

L'exemple suivant illustre la présente invention et ne doit être considéré en aucune manière comme limitant l'invention.

### EXEMPLES

On prépare un produit de coiffage sous forme de spray aérosol, comportant au plus 55 % de composés organiques volatils, à partir des ingrédients suivants. Les quantités sont indiquées en % en poids :
- Luviset® Si PUR vendu par BASF 6,5 %
- Viscophobe® DB 1000 vendu par Union Carbide 1 %
   Aminométhyl-propanol 0,1 %
   Ethanol 17 %
   Diméthyléther 35 %
   Eau qsp 100 %

On pulvérise ce produit de coiffage sur la chevelure et on met en forme la coiffure, on obtient un bon maintien de la coiffure.

## Revendications

1. Composition de traitement cosmétique des cheveux comprenant, dans un milieu cosmétiquement acceptable, au moins un polyuréthane fixant non associatif et au moins un polyuréthane associatif anionique ou non ionique, conditionnée dans un dispositif aérosol en présence d'un agent propulseur.

2. Composition de traitement cosmétique des cheveux selon la revendication 1, **caractérisée en ce que** le polyuréthane fixant non associatif comprend un motif répétitif de base répondant à la formule générale (I) :
- O - B - O - CO - NH - R - NH - CO - (I)
dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes alkylènes de type aromatique, aliphatique en C₁ à C₂₀, cycloaliphatique en C₁ à C₂₀, ces groupes étant substitués ou non.

3. Composition de traitement cosmétique des cheveux selon la revendication 1, **caractérisée en ce que** le polyuréthane fixant non associatif comprend un motif répétitif de base répondant à la formule générale (II) :
- O - P - O - CO - NH - R - NH - CO - (II)
dans laquelle :
- P est un segment polysiloxanique, et
- R est un groupe divalent choisi parmi les groupes alkylènes de type aromatique, aliphatique en C₁ à C₂₀, cycloaliphatique en C₁ à C₂₀, ces groupes étant substitués ou non.

4. Composition de traitement cosmétique des cheveux selon la revendication 1 ou 2, **caractérisée en ce que** le polyuréthane fixant non associatif est le copolymère acide diméthylolpropionique/isophoronediisocyanate/néopentylglycol/polyesterdiols.

5. Composition de traitement cosmétique des cheveux selon la revendication 1 ou 3, **caractérisée en ce que** le polyuréthane fixant non associatif est le copolymère acide diméthylolpropionique/isophoronediisocyanate/néopentylglycol/polyesterdiols/diamine siliconée.

6. Composition de traitement cosmétique des cheveux selon l'une quelconque des revendications précédentes, **caractérisée en ce que** le polyuréthane associatif anionique est un terpolymère acrylique comprenant :
a) environ 20 à 70% en poids, de préférence 25 à 55% en poids, d'un acide carboxylique à insaturation α, β-monoéthylénique ;
b) environ 20 à 80% en poids, de préférence 30 à 65% en poids, d'un monomère à insaturation monoéthylénique non tensioactif différent de a) et
c) environ 0,5 à 60% en poids, de préférence 10 à 50% en poids, d'un monomère uréthane non-ionique qui est le produit de réaction d'un tensioactif non-ionique monohydrique avec un monoisocyanate à insaturation monoéthylénique.

7. Composition de traitement cosmétique des cheveux selon la revendication 6, **caractérisée en ce que** le polyuréthane associatif anionique est un copopolymère acide méthacrylique/méthacrylate de méthyle/méthylstyrène-isopropylisocyanate/alcool béhénylique polyéthoxylé comportant 40 motifs éthoxy.

8. Composition de traitement cosmétique des cheveux selon l'une quelconque des revendications 1 à 5, **caractérisée en ce que** le polyuréthane associatif non ionique est un polyuréthane-polyéther susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

9. Composition de traitement cosmétique des cheveux selon la revendication 8, **caractérisée en ce que** le polyuréthane associatif non ionique est un polycondensat de polyéthylèneglycol à 150 ou 180 moles d'oxyde d'éthylène, d'alcool stéarylique et de méthylène-bis(4-cyclohexylisocyanate) ou un polycondensat de polyéthylèneglycol à 150 ou 180 moles d'oxyde d'éthylène, d'alcool décylique et de méthylène-bis(4-cyclohexylisocyanate).

10. Composition de traitement cosmétique des cheveux selon l'une quelconque des revendications précédentes, **caractérisée en ce que** le polyuréthane fixant non associatif est présent en une quantité de 0,1 à 20 % en poids, de préférence de 0,5 à 10 % en poids et mieux encore de 1 à 5 % en poids par rapport au poids total de la composition.

11. Composition de traitement cosmétique des cheveux selon l'une quelconque des revendications précédentes, **caractérisée en ce que** le polyuréthane associatif anionique ou non ionique est présent en une quantité de 0,1 à 10 % en poids, de préférence de 0,2 à 8 % en poids et mieux encore de 0,5 à 5 % en poids par rapport au poids total de la composition.

12. Composition de traitement cosmétique des cheveux selon l'une quelconque des revendications précédentes, **caractérisée en ce que** l'agent propulseur est choisi parmi les gaz hydrocarbonés, les gaz fluorés, l'azote, l'air, le dioxyde de carbone, le diméthyléther et leurs mélanges.

13. Composition de traitement cosmétique des cheveux selon la revendication 12, **caractérisée en ce que** l'agent propulseur est choisi parmi le diméthyléther, les gaz hydrocarbonés et leurs mélanges.

14. Composition de traitement cosmétique des cheveux selon l'une quelconque des revendications précédentes, **caractérisée en ce que** l'agent propulseur est présent en une quantité de 2 à 90 % en poids, de préférence de 5 à 80 % en poids par rapport au poids total de la composition.

15. Composition de traitement cosmétique des cheveux selon l'une quelconque des revendications précédentes, **caractérisée en ce que** le milieu cosmétiquement acceptable est constitué uniquement par de l'eau ou par un mélange d'eau et d'un solvant cosmétiquement acceptable.

16. Composition de traitement cosmétique des cheveux selon la revendication 15, **caractérisée en ce que** le solvant cosmétiquement acceptable est choisi parmi les alcools inférieurs en C₁-C₄, les alkylènepolyols, les éthers de polyols et leurs mélanges.

17. Composition de traitement cosmétique des cheveux selon la revendication 15 ou 16, **caractérisée en ce que** le solvant cosmétiquement acceptable est choisi parmi l'éthanol, l'isopropanol, le tertio-butanol, le n-butanol et le propylèneglycol.

18. Composition de traitement cosmétique des cheveux selon l'une quelconque des revendications précédentes, **caractérisée en ce qu'**elle comprend en outre des additifs choisis parmi d'autres polymères fixants, les polymères cationiques, amphotères ou zwitteroniques, d'autres polymères anioniques ou non-ioniques, les agents épaississants, les nacrants, les opacifiants, les filtres UV, les sucres, les parfums, les huiles minérales, végétales et/ou synthétiques, les esters d'acides gras, les colorants, les silicones volatiles ou non, organomodifiées ou non, cycliques ou acycliques, ramifiées ou non, les particules minérales ou organiques, naturelles ou synthétiques, les conservateurs et les agents de stabilisation du pH.

19. Composition de traitement cosmétique des cheveux selon l'une quelconque des revendications précédentes, **caractérisée en ce qu'**elle se présente sous la forme d'une mousse, d'un gel, d'un spray ou d'une laque.

20. Composition de traitement cosmétique des cheveux selon l'une quelconque des revendications précédentes, **caractérisée en ce qu'**elle se présente sous la forme d'un produit de fixation de la coiffure.

21. Procédé de traitement cosmétique des cheveux, **caractérisé en ce que** l'on applique sur les cheveux une composition de traitement cosmétique des cheveux selon l'une quelconque des revendications précédentes.

22. Utilisation d'une composition selon l'une quelconque des revendications 1 à 20, comme produit de coiffage non rincé.

## Claims

1. Cosmetic hair treatment composition comprising, in a cosmetically acceptable medium, at least one nonassociative fixing polyurethane and at least one anionic or nonionic associative polyurethane, packaged in an aerosol device in the presence of a propellant.

2. Cosmetic hair treatment composition according to Claim 1, **characterized in that** the nonassociative fixing polyurethane comprises a repeating base unit corresponding to the general formula (I):
- O - B - O - CO - NH - R - NH - CO - (I)
in which:
- B is a divalent C₁ to C₃₀ hydrocarbon group, this group possibly being substituted with a group comprising one or more carboxylic acid functions and/or one or more sulfonic acid functions, said carboxylic acid and/or sulfonic acid functions being in free form or else partially or totally neutralized with a mineral or organic base, and
- R is a divalent group chosen from alkylene groups of aromatic type, C₁ to C₂₀ aliphatic groups and C₁ to C₂₀ cycloaliphatic groups, these groups possibly being substituted.

3. Cosmetic hair treatment composition according to Claim 1, **characterized in that** the nonassociative fixing polyurethane comprises a repeating base unit corresponding to the general formula (II):
- O - P - O - CO - NH - R - NH - CO - (II)
in which:
- P is a polysiloxane segment, and
- R is a divalent group chosen from alkylene groups of aromatic type, C₁ to C₂₀ aliphatic groups and C₁ to C₂₀ cycloaliphatic groups, these groups possibly being substituted.

4. Cosmetic hair treatment composition according to Claim 1 or 2, **characterized in that** the nonassociative fixing polyurethane is the dimethylolpropionic acid/isophorone diisocyanate/neopentyl glycol/polyester diols copolymer.

5. Cosmetic hair treatment composition according to Claim 1 or 3, **characterized in that** the nonassociative fixing polyurethane is the dimethylolpropionic acid/isophorone diisocyanate/neopentyl glycol/polyester diols/diamino silicone copolymer.

6. Cosmetic hair treatment composition according to any one of the preceding claims, **characterized in that** the anionic associative polyurethane is an acrylic terpolymer comprising:
a) about 20% to 70% by weight, preferably 25% to 55% by weight, of a carboxylic acid containing α, β-monoethylenic unsaturation;
b) about 20% to 80% by weight, preferably 30% to 65% by weight, of a nonsurfactant monomer containing monoethylenic unsaturation, which is different than a), and
c) about 0.5% to 60% by weight, preferably 10% to 50% by weight, of a nonionic urethane monomer which is the product of reaction of a monohydric nonionic surfactant with a monoisocyanate containing monoethylenic unsaturation.

7. Cosmetic hair treatment composition according to Claim 6, **characterized in that** the anionic associative polyurethane is a methacrylic acid/methyl methacrylate/methylstyreneisopropyl isocyanate/ polyethoxylated behenyl alcohol copolymer comprising 40 ethoxy units.

8. Cosmetic hair treatment composition according to any one of Claims 1 to 5, **characterized in that** the nonionic associative polyurethane is a polyurethane-polyether obtainable by polycondensation of at least three compounds comprising (i) at least one polyethylene glycol comprising from 150 to 180 mol of ethylene oxide, (ii) stearyl alcohol or decyl alcohol, and (iii) at least one diisocyanate.

9. Cosmetic hair treatment composition according to Claim 8, **characterized in that** the nonionic associative polyurethane is a polycondensate of polyethylene glycol containing 150 or 180 mol of ethylene oxide, of stearyl alcohol and of methylenebis-(4-cyclohexyl isocyanate) or a polycondensate of polyethylene glycol containing 150 or 180 mol of ethylene oxide, of decyl alcohol and of methylenebis(4-cyclohexyl isocyanate).

10. Cosmetic hair treatment composition according to any one of the preceding claims, **characterized in that** the nonassociative fixing polyurethane is present in an amount from 0.1% to 20% by weight, preferably from 0.5% to 10% by weight and better still from 1% to 5% by weight, relative to the total weight of the composition.

11. Cosmetic hair treatment composition according to any one of the preceding claims, **characterized in that** the anionic or nonionic associative polyurethane is present in an amount from 0.1% to 10% by weight, preferably from 0.2% to 8% by weight and better still from 0.5% to 5% by weight, relative to the total weight of the composition.

12. Cosmetic hair treatment composition according to any one of the preceding claims, **characterized in that** the propellant is chosen from hydrocarbon gases, fluorinated gases, nitrogen, air, carbon dioxide and dimethyl ether, and mixtures thereof.

13. Cosmetic hair treatment composition according to Claim 12, **characterized in that** the propellant is chosen from dimethyl ether and hydrocarbon gases, and mixtures thereof.

14. Cosmetic hair treatment composition according to any one of the preceding claims, **characterized in that** the propellant is present in an amount from 2% to 90% by weight and preferably from 5% to 80% by weight, relative to the total weight of the composition.

15. Cosmetic hair treatment composition according to any one of the preceding claims, **characterized in that** the cosmetically acceptable medium consists solely of water or of a mixture of water and a cosmetically acceptable solvent.

16. Cosmetic hair treatment composition according to Claim 15, **characterized in that** the cosmetically acceptable solvent is chosen from C₁-C₄ lower alcohols, alkylene polyols and polyol ethers, and mixtures thereof.

17. Cosmetic hair treatment composition according to Claim 15 or 16, **characterized in that** the cosmetically acceptable solvent is chosen from ethanol, isopropanol, tert-butanol, n-butanol and propylene glycol.

18. Cosmetic hair treatment composition according to any one of the preceding claims, **characterized in that** it also comprises additives chosen from other fixing polymers, cationic, amphoteric or zwitterionic polymers, other anionic or nonionic polymers, thickeners, nacreous agents, opacifiers, UV screening agents, sugars, fragrances, mineral, plant and/or synthetic oils, fatty acid esters, colorants, volatile or nonvolatile, cyclic or acyclic, branched or unbranched silicones, which may or may not be organomodified, natural or synthetic, mineral or organic particles, preserving agents and pH stabilizers.

19. Cosmetic hair treatment composition according to any one of the preceding claims, **characterized in that** it is in the form of a mousse, a gel, a spray or a lacquer.

20. Cosmetic hair treatment composition according to any one of the preceding claims, **characterized in that** it is in the form of a hairstyle fixing product.

21. Cosmetic hair treatment process, **characterized in that** a cosmetic hair treatment composition according to any one of the preceding claims is applied to the hair.

22. Use of a composition according to any one of Claims 1 to 20, as a leave-in styling product.

## Patentansprüche

1. Zusammensetzung zur kosmetischen Behandlung der Haare, die in einem kosmetisch annehmbaren Medium mindestens ein nichtassoziatives festigendes Polyurethan und mindestens ein anionisches oder nichtionisches assoziatives Polyurethan enthält und in einer Aerosolvorrichtung in Gegenwart eines Treibmittels abgepackt ist.

2. Zusammensetzung zur kosmetischen Behandlung der Haare nach Anspruch 1, **dadurch gekennzeichnet, daß** das nichtassoziative festigende Polyurethan eine Grundwiederholungseinheit der allgemeinen Formel (I) enthält:
-O-B-O-CO-NH-R-NH-CO- (I)
worin:
- B für eine zweiwertige C₁- bis C₃₀-Kohlenwasserstoffgruppe steht, die gegebenenfalls durch eine Gruppe mit einer oder mehreren Carbonsäurefunktionen und/oder einer oder mehreren Sulfonsäurefunktionen substituiert ist, wobei die Carbonsäure- und/oder Sulfonsäurefunktionen in freier Form oder auch teilweise oder vollständig durch eine anorganische oder organische Base neutralisiert vorliegen, und
- R für eine zweiwertige Gruppe steht, die unter gegebenenfalls substituierten aromatischen Alkylengruppen, gegebenenfalls substituierten aliphatischen C₁- bis C₂₀-Alkylengruppen und gegebenenfalls substituierten cycloaliphatischen C₁- bis C₂₀-Alkylengruppen ausgewählt ist.

3. Zusammensetzung zur kosmetischen Behandlung der Haare nach Anspruch 1, **dadurch gekennzeichnet, daß** das nichtassoziative festigende Polyurethan eine Grundwiederholungseinheit der allgemeinen Formel (II) enthält:
-O-P-O-CO-NH-R-NH-CO- (II)
worin:
- P für ein Polysiloxansegment steht und
- R für eine zweiwertige Gruppe steht, die unter gegebenenfalls substituierten aromatischen Alkylengruppen, gegebenenfalls substituierten aliphatischen C₁- bis C₂₀-Alkylengruppen und gegebenenfalls substituierten cycloaliphatischen C₁- bis C₂₀-Alkylengruppen ausgewählt ist.

4. Zusammensetzung zur kosmetischen Behandlung der Haare nach Anspruch 1 oder 2, **dadurch gekennzeichnet, daß** es sich bei dem nichtassoziativen festigenden Polyurethan um das Copolymer von Dimethylpropionsäure, Isophorondiisocyanat, Neopentylglykol und Polyesterdiolen handelt.

5. Zusammensetzung zur kosmetischen Behandlung der Haare nach Anspruch 1 oder 3, **dadurch gekennzeichnet, daß** es sich bei dem nichtassoziativen festigenden Polyurethan um das Copolymer von Dimethylpropionsäure, Isophorondiisocyanat, Neopentylglykol, Polyesterdiolen und Silikondiamin handelt.

6. Zusammensetzung zur kosmetischen Behandlung der Haare nach einem der vorhergehenden Ansprüche, **dadurch gekennzeichnet, daß** es sich bei dem anionischen assoziativen Polyurethan um ein Acrylterpolymer, enthaltend
a) etwa 20 bis 70 Gew.-% und vorzugsweise 25 bis 55 Gew.-% einer α,β-ungesättigten Carbonsäure,
b) etwa 20 bis 80 Gew.-% und vorzugsweise 30 bis 65 Gew.-% eines nicht oberflächenaktiven monoethylenisch ungesättigten Monomers, das von a) verschieden ist, und
c) etwa 0,5 bis 60 Gew.-% und vorzugsweise 10 bis 50 Gew.-% eines nichtionischen Urethanmonomers, bei dem es sich um das Produkt der Umsetzung eines monohydroxylierten nichtionischen Tensids mit einem monoethylenisch ungesättigten Monoisocyanat handelt,handelt.

7. Zusammensetzung zur kosmetischen Behandlung der Haare nach Anspruch 6, **dadurch gekennzeichnet, daß** es sich bei dem anionischen assoziativen Polyurethan um ein Copolymer von Methacrylsäure, Methylmethacrylat, Methylstyrolisopropylisocyanat und polyoxyethyliertem Behenylalkohol mit 40 Ethoxyeinheiten handelt.

8. Zusammensetzung zur kosmetischen Behandlung der Haare nach einem der Ansprüche 1 bis 5, **dadurch gekennzeichnet, daß** es sich bei dem nichtionischen assoziativen Polyurethan um einen Polyurethanpolyether handelt, der durch Polykondensation von mindestens drei Verbindungen, umfassend (i) mindestens ein Polyethylenglykol mit 150 bis 180 mol Ethylenoxid, (ii) Stearylalkohol oder Decylalkohol und (iii) mindestens ein Diisocyanat, erhältlich ist.

9. Zusammensetzung zur kosmetischen Behandlung der Haare nach Anspruch 8, **dadurch gekennzeichnet, daß** es sich bei dem nichtionischen assoziativen Polyurethan um ein Polykondensat von Polyethylenglykol mit 150 bis 180 mol Ethylenoxid, Stearylalkohol und Methylenbis(4-cyclohexylisocyanat) oder ein Polykondensat von Polyethylenglykol mit 150 bis 180 mol Ethylenoxid, Decylalkohol und Methylen-bis(4-cyclohexylisocyanat) handelt.

10. Zusammensetzung zur kosmetischen Behandlung der Haare nach einem der vorhergehenden Ansprüche, **dadurch gekennzeichnet, daß** das nichtassoziative festigende Polyurethan in einer Menge von 0,1 bis 20 Gew.-%, vorzugsweise von 0,5 bis 10 Gew.-% und noch besser von 1 bis 5 Gew.-%, bezogen auf das Gesamtgewicht der Zusammensetzung, vorliegt.

11. Zusammensetzung zur kosmetischen Behandlung der Haare nach einem der vorhergehenden Ansprüche, **dadurch gekennzeichnet, daß** das anionische oder nichtionische assoziative Polyurethan in einer Menge von 0,1 bis 10 Gew.-%, vorzugsweise von 0,2 bis 8 Gew.-% und noch besser von 0,5 bis 5 Gew.-%, bezogen auf das Gesamtgewicht der Zusammensetzung, vorliegt.

12. Zusammensetzung zur kosmetischen Behandlung der Haare nach einem der vorhergehenden Ansprüche, **dadurch gekennzeichnet, daß** das Treibmittel unter Kohlenwasserstoffgasen, fluorhaltigen Gasen, Stickstoff, Luft, Kohlendioxid, Dimethylether und Gemischen davon ausgewählt ist.

13. Zusammensetzung zur kosmetischen Behandlung der Haare nach Anspruch 12, **dadurch gekennzeichnet, daß** das Treibmittel unter Dimethylether, Kohlenwasserstoffgasen und Gemischen davon ausgewählt ist.

14. Zusammensetzung zur kosmetischen Behandlung der Haare nach einem der vorhergehenden Ansprüche, **dadurch gekennzeichnet, daß** das Treibmittel in einer Menge von 2 bis 90 Gew.-% und vorzugsweise von 5 bis 80 Gew.-%, bezogen auf das Gesamtgewicht der Zusammensetzung, vorliegt.

15. Zusammensetzung zur kosmetischen Behandlung der Haare nach einem der vorhergehenden Ansprüche, **dadurch gekennzeichnet, daß** das kosmetisch annehmbare Medium einzig und allein aus Wasser oder einem Gemisch von Wasser und einem kosmetisch annehmbaren Lösungsmittel besteht.

16. Zusammensetzung zur kosmetischen Behandlung der Haare nach Anpruch 15, **dadurch gekennzeichnet, daß** das kosmetisch annehmbare Lösungsmittel unter niederen Alkoholen mit 1 bis 4 Kohlenstoffatomen, Alkylenpolyolen, Polyolethern und Gemischen davon ausgewählt ist.

17. Zusammensetzung zur kosmetischen Behandlung der Haare nach Anpruch 15 oder 16, **dadurch gekennzeichnet, daß** das kosmetisch annehmbare Lösungsmittel unter Ethanol, Isopropanol, tert-Butanol, n-Butanol und Propylenglykol ausgewählt ist.

18. Zusammensetzung zur kosmetischen Behandlung der Haare nach einem der vorhergehenden Ansprüche, **dadurch gekennzeichnet, daß** sie weiterhin mindestens einen Zusatzstoff enthält, der unter anderen festigenden Polymeren, kationischen, amphoteren oder zwitterionischen Polymeren, anderen anionischen oder nichtionischen Polymeren, Verdickern, Perlglanzmitteln, Trübungsmitteln, UV-Filtern, Zuckern, Duftstoffen, mineralischen, pflanzlichen und/oder synthetischen Ölen, Fettsäureestern, Farbmitteln, flüchtigen oder nichtflüchtigen, gegebenenfalls organisch modifizierten, cyclischen oder acyclischen, gegebenenfalls verzweigten Silikonen, natürlichen oder synthetischen, anorganischen oder organischen Teilchen, Konservierungsmitteln und pH-Stabilisatoren ausgewählt ist.

19. Zusammensetzung zur kosmetischen Behandlung der Haare nach einem der vorhergehenden Ansprüche, **dadurch gekennzeichnet, daß** sie in Form eines Schaums, eines Gels, eines Sprays oder eines Lacks vorliegt.

20. Zusammensetzung zur kosmetischen Behandlung der Haare nach einem der vorhergehenden Ansprüche, **dadurch gekennzeichnet, daß** sie in Form eines Frisurfestigerprodukts vorliegt.

21. Verfahren zur kosmetischen Behandlung der Haare, **dadurch gekennzeichnet, daß** man auf die Haare eine Zusammensetzung zur kosmetischen Behandlung der Haare gemäß einem der vorhergehenden Ansprüche aufbringt.

22. Verwendung einer Zusammensetzung gemäß einem der Ansprüche 1 bis 20 als nicht auszuspülendes Frisierprodukt.
